# EUROPEAN PATENT SPECIFICATION

(11) **EP 4 062 757 B1**
(45) Date of publication and mention of the grant of the patent: **07.02.2024**
(21) Application number: 21165414.0
(22) Date of filing: 26.03.2021
(51) Int. Cl.: A01K 67/033

(54) **SYSTEM FOR BREEDING INSECTS AT THEIR IMAGINAL STAGE**
SYSTEM ZUR AUFZUCHT VON INSEKTEN IN DEREN ADULTFORM
SYSTÈME POUR L'ÉLEVAGE D'INSECTES À LEUR STADE IMAGINAL

(43) Date of publication of application: 28.09.2022
(73) Proprietor: Nasekomo B.V., 1075 HP Amsterdam (NL)
(72) Inventor: BOLARD, Marc Louis Raymond, 1407 Sofia (BG)
(74) Representative: Fidal Innovation

(56) References cited:
- WO-A1-2013/166590
- CN-A- 105 340 843
- US-A1- 2011 296 756
- US-B2- 8 733 284

## Description

### FIELD OF THE INVENTION

The present invention relates to the field of insect breeding, in particular the production of Dipteran insects.

More specifically, the invention relates to an automated cycle for controlling the reproduction of adult insects, between the pupation stage and the death of these insects, as well as the collection of their eggs.

### TECHNOLOGICAL BACKGROUND

Insects, in particular certain species, can constitute a source of products or of raw materials, in particular for animal feed or human foodstuffs. They are particularly well-suited to converting organic materials, such as commercial or residential waste products, or low quality biomass into foodstuff of high nutritional quality, with high protein content.

The optimization of the insect breeding is in consequence of paramount importance to ensure the sustainable food needs of our planet.

In the field of insect rearing, it is known to create devices or even farms in which the different stages of insect life are managed at different places, at each of which the environmental conditions are optimized for a given stage.

WO2019/053456 A1 is an example of a modular system for fly breeding, in which the life of insects is subdivided into discrete stages forming a cycle. A batch of insects, or a combination of batches of insects, goes through these different stages, for each of which the humidity, temperature and lighting conditions are controlled. In particular, the stages are distinguished: "egg growth chamber", "larval chamber", "pupation chamber", "emergence box" and "reproduction chamber".

However, this device does not disclose any details of what occurs in the "reproduction chamber".

WO2016/011541 A1 discloses a mating chamber for insects, in particular Hermetica Illucens, comprising several successive automated steps, all identical to each other. A given pupation chamber enters from one side of the chamber, advances one stage per day so that it replaces the pupation chamber which precedes it and it is replaced by the pupation chamber which follows. After n days spent in the mating chamber, the pupation chamber exits the mating chamber. This device is only operable in a FIFO mode, and it does neither give any detail of the conditions or the operations occurring in the mating chamber.

US10,405,528B2 discloses an automated farm for insect breeding, comprising a plurality of interchangeable stations, among : feeding the insects, providing them with water, renewing the substrate, identifying insects having a disease, killing surplus, diseased and/or contaminated insects, etc. However, this document doesn't disclose a cycle including one or more of these steps, this cycle being designed in order to optimize the mating step of the insects.

US2011/296756 discloses a system for breeding insects in view of their reproduction without automation.

WO2013/166590 A1 discloses an automated system for breeding insects at their imaginal stage, comprising at least one mating cage, a filling system to fill the at least one mating cage with a population of newly emerged insects, a provision system to provide conditions within at least one mating cage suitable for promoting mating and/or oocytes maturation and/or ovipositing, and a terminating system to stop ovipositing during a third time interval, comprising a mating chamber in which newly emerged insects can continuously pass from a pupation chamber. The optimisation of the mating is not considered but not the optimisation of the egg yield.

Hence, this invention aims at providing a fully automated mating cycle in order to optimize the mating step and the collect of the eggs and permitting specific operations or specific tuning at each given step of the cycle if needed.

### SUMMARY OF THE INVENTION

The disclosure relates to an automated process for breeding insects at their imaginal stage in at least one mating cage which does not form part of the invention. This process comprises the steps of:
- a: providing at least one emergence chamber comprising newly emerged adults and having one door or hatch the closing and opening of which is automated;
- b: connecting at least one mating cage through the at least one door or hatch to at least one emergence chamber comprising a door or a hatch the closing and opening of which is automated;
- c: filling the at least one mating cage with a population of newly emerged insects during a first time interval T1 and automatically closing successively or simultaneously the at least one door or hatch of the at least one mating cage and the at least one door or hatch of the at least one emergence chamber once a wished population has entered the mating cage and or/after a predefined connection time;
- d: providing conditions within the at least one of the at least one mating cage suitable for promoting mating and/or oocytes maturation and/or ovipositing during a second time interval T2;
- e: stopping ovipositing during a third time interval T3, the time between the beginning of said step c. and the beginning of said step e. being shorter than the life expectancy of the adult insects after emergence.

Stopping ovipositing makes it possible to optimize the ovipositing yield, since most of the ovipositing occur in the early days of female adult life. The ovipositing yield grows indeed much faster in the first days of life of the adults than at the end of their life.

This stopping step also makes it possible to build a periodic cycle that can be easily managed in an automated manner.

In a particular embodiment, during step d of the process, ovipositing devices are provided and/or collected without opening said mating cage 1.

This prevents adults loss and contributes to optimize the egg yield.

In a particular embodiment, the process also comprises a step f. of cleaning and emptying one mating cage during a fourth time interval T4 and optionally one or more steps g. of storing the mating cage during a fifth time interval T5 after step e. and that after the last of steps f. and g., said mating cage is suitable for a new step b.

This makes it possible to reuse the same mating cage periodically in good sanitary conditions.

In a particular embodiment, the process comprises at least one additional step h. of providing conditions within the mating cage suitable for promoting mating during and/or oocytes maturation and/or ovipositing during a sixth time interval T6 between said steps d. and e.. The conditions of step h may differ from the conditions of step d.

This makes it possible to fine-tune the conditions either for mating, or for oocytes maturation or for ovipositing, or for a combination of two of these stages, and as a consequence, improve the egg-yield.

In a particular embodiment, the time between the beginning of step c. and the beginning of step e. is shorter than the life expectancy of the adult insects after emergence.

In a particular embodiment, step e starts once one of the following threshold is met : the percentage of dead adults meets a first threshold, the percentage of females having oviposited meets a second threshold, the egg yield meets a third threshold.

These two embodiments make it possible to optimize the egg-yield by not waiting for the death of the adults.

In a particular embodiment, at least two of the time intervals of the process are equal.

This makes it possible to synchronize several steps of the process.

In a particular embodiment, at least two steps of the process take place at two different places, and the mating cage is moved between the two different places in between the two steps.

In this case, several mating cages can be at several steps of the process at the same time.

In a particular embodiment, at least one condition of at least one of steps d. or h. are chosen in the list {ambient temperature, relative humidity, lighting, feeding, watering}.

These conditions are known to inhibit or enhance ovipositing and/or oocyte maturation and/or mating. In consequence, adjustment of these conditions at each step helps improve the egg-yield.

In a particular embodiment, the population filling the mating cage is selected according to one or more criteria among {sex ratio, phenotype, genotype, size of the larvae at a given date, sexual maturity, age}.

This makes it possible to control, or even homogenize, the quality of the eggs collected and to optimize the egg-yield.

The invention relates to an automated system for breeding insects at their imaginal stage, comprising :
- at least one mating cage having at least one door or hatch (12) the closing and opening of which is automated and through which said mating cage (1) can be connected to at least one emergence chamber having at least one door or hatch the closing and opening of which is automated;
- a filling system to fill the at least one mating cage with a population of newly emerged insects during a first time interval T1 and to automatically close successively or simultaneously the at least one door or hatch of the mating cage and the at least one door or hatch of at least one emergence chamber once a wished population has entered the mating cage and or/after a predefined connection time;
- a provision system to provide conditions within the at least one mating cage suitable for promoting mating and/or oocytes maturation and/or ovipositing during a second time interval T2;
- a terminating system to stop ovipositing during a third time interval T3.

In a particular embodiment, the at least one door of the at least one mating cage of the automated system comprises an automated device for its closing and opening. The at least one mating cage is provided with removable ovipositing devices. It has at least two discrete positions.

The automated system further comprises a device for moving the at least one mating cage from at least one of the at least two discrete positions to at least another of the at least two discrete positions, each discrete position being equipped to automatically perform at least one step of the automated process for breeding insects at their imaginal stage.

The door makes it possible to automatically connect the mating cage with an emergence chamber.

The discrete positions allow the setting of different conditions at each position, specifically adjusted for a given step, rather than changing theses conditions at the same location for a mating cage remaining there.

In a particular embodiment of the automated system, a tracking device is provided for each of the at least one mating cage.

In a particular embodiment, the automated system further comprises a controller and a tracking device is provided for each mating cage. The controller receives tracking data from at least one mating cage and controls the moving of the mating cage based on the tracking data.

This allows for example to control automatedly the filling of the mating cage, and the moving of a mating cage from one step to another.

In a particular embodiment of the automated system, the device for moving a mating cage is a conveyor belt or a rail or a suspended conveyor rail or an automated guided vehicle.

In a particular embodiment of the automated system, each mating cage is provided with an automated device for counting the adult population.

This allows the monitoring of the filling of the mating cage.

In a particular embodiment of the automated system, the automated device for counting the population comprises a weight sensor.

This allows an easy monitoring of the filling of the mating cage, for example when the mating cage is suspended with a hook.

In a particular embodiment of the automated system, the automated device for closing and opening the door of the at least one mating cage receives a signal from said automated device for counting the adult population that triggers the closing and/or opening of said door.

In this case, the filling of the mating cage can be completely automated.

In a particular embodiment of the automated system, the at least one mating cage is made of a mesh made of metal or fabric or plastic.

This allows ventilation, cleaning and oviposition on ovipositing devices on the outer side of the mating cage.

In a particular embodiment of the automated system, at least one removable ovipositing device is provided for each of the at least one mating cage. The removable ovipositing device can be fixed on the outer side of said mating cage.

In this case, the collection of the ovipositing devices can occur at any time, it is easier and there is no loss of adults during such an operation.

In a particular embodiment of the automated system, a tracking device is provided for each of the removable ovipositing devices.

In a particular embodiment, the system further comprises a controller receiving data from at least one tracking device of an ovipositing device and controls the fixing of the ovipositing device based on the tracking data received from the ovipositing device.

This allows an automated collection of the eggs and the monitoring of it. In particular, the egg-yield can be studied at each collection operation and in consequence, the collection can be optimized.

The invention also relates to a system comprising a system an automated system for breeding insects at their imaginal stage coupled with a system for breeding insects at the egg stage and or the pupation stage.

A system for breeding insects at the pupation stage produces the population for filling the mating cage according to the invention.

The eggs collected in a system for breeding insects at their imaginal stage according to the invention can be used in a system for breeding insects at the egg stage.

### BRIEF DESCRIPTION OF THE DRAWINGS

Embodiments of the invention will be described below, in relation to the following drawings
Fig. 1 is a view of a mating cage with which the process can be implemented.
Fig. 2 is a view of an ovipositing device.
Fig. 3 shows a mating cycle according to one embodiment.

On the drawings, the same reference signs show the same or similar objects.

### DETAILED DESCRIPTION

The invention deals with the optimization of insects rearing.

In general, Dipteran insects, such as the Black Soldier Fly (Hermetica Illucens), can be bred according to the invention described below, but other insects with a flying stage could be considered.

Black Soldier Flies are particularly well adapted for farming, since they don't bite nor sting, they aren't likely to function as a vector for diseases since they aren't attracted to human environments, and they have a high rate of conversion of biomass into protein.

The life cycle of an insect comprises stages : the egg stage, the larval stage, the nymph stage in the case of Endopterygota, the adult or imaginal stage (adult insect and imago being synonymous).

For Dipteran insects, the stage that precedes the adult stage, or imaginal stage, is called "pupal stage" rather than nymph stage. The pupal stage follows the larval stage and the transition between the pupal stage and the adult stage is called the emergence.

The disclosure concerns more specifically a process for managing the imaginal phase, from the emergence until the death of the imago, which does not form part of the invention.

According to one embodiment, the process comprises at least the following steps :
- Step 1 : an empty mating cage 1 is connected to at least one emergence chamber, so as to be filled with a population of newly emerged adults.
- Step 2 : once filled, the mating cage 1 undergoes a first lighting cycle designed to promote the insects mating and oocyte maturation ; the mating cage 1 is equipped with one ore more ovipositing devices 2 on which the gravid females can oviposit their fertilized eggs.
- Step 3 : the mating cage 1 undergoes a second lighting cycle designed to promote the insects mating and the oocyte maturation ; the mating cage 1 is equipped with one ore more ovipositing devices 2 on which the gravid females can oviposit their fertilized eggs. Some ovipositing devices 2 are collected at the beginning of or during or at the end of step 3;
- Step 4 : the mating cage 1 is equipped with one ore more ovipositing devices 2 on which the gravid females can oviposit their fertilized eggs. Some ovipositing devices 2 are collected at the beginning of or during or at the end of step 4. The mating cage undergoes a third lighting cycle designed to promote oviposition ;
- Step 4' (optional) : the mating cage 1 is equipped with one ore more ovipositing devices 2 on which the gravid females can oviposit their fertilized eggs. Some ovipositing devices 2 are collected at the beginning of or during or at the end of step 4'. Mating cage 1 undergoes a fourth lighting cycle designed to promote oviposition.
- Step 5 : After step 4 or step 4', the adults that are not already dead are killed before their natural death ;
- Step 6 : the mating cage 1 is cleaned before entering a new cycle.

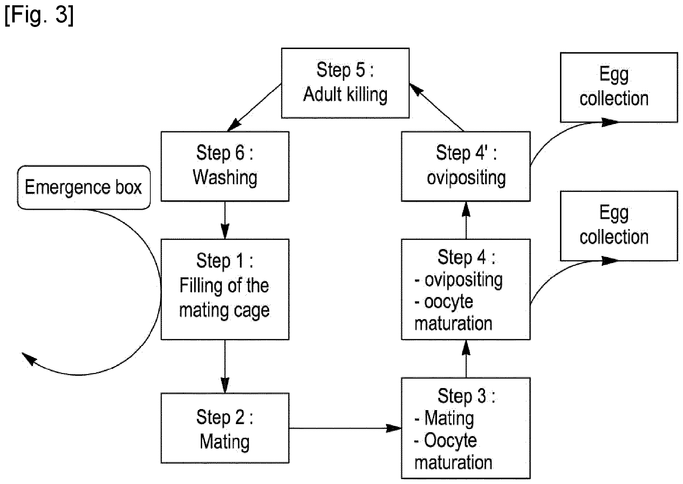

An exemplary embodiment of the process is shown on figure 3.

The process can be implemented on a device comprising at least one mating cage (or mating chamber) 1, in which any given adult undergoes step 1 to step 5.

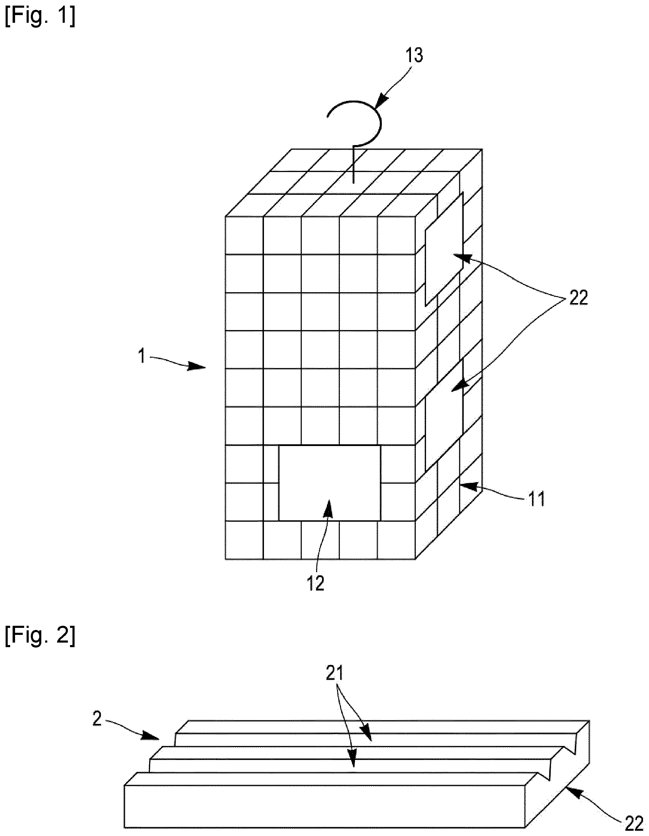

In an exemplary but non limitative embodiment, in relation to figure 1, a mating cage (or mating chamber) 1 is a mesh parallelepipedic box. The dimensions of the box can be adapted to the insect being reared.

For example, since adult black fly soldier fly less than 10% of the time, the height of the box can be larger than the width and length, in order to have both a high surface-to-volume ratio, since mating takes place on the surface of the box, and a sufficient height for the mating dance (or lekking behavior), which is mainly vertical and precedes mating.

In one embodiment, a mating chamber 1 has a length and a width of the same order of magnitude and a height around 1 to 10 times the length or the width.

The mesh 11 can be made of metal or plastic or fabric or fiberglass for example. The size of the mesh is chosen so as to prevent adults from escaping and to permit oviposition on a device outside the box. It will be understood that the scale was not respected for the mesh size on figure 1, in order to make figure 1 more readable.

Optionally, part or all of the side walls and/or of the bottom of the mating cage 1 are opaque and made of the same or a different material than the other parts of the walls. This disposition helps to attract the flies to a brighter part of the cage when the light inside the box is on.

In one embodiment, at least two steps of the process are operated at two different places.

In one embodiment, each step of the process is operated at a different location.

In one embodiment, the process is automated. For this purpose, a mechanism may be provided for passing a mating chamber from one location to the following.

For example, the mating chamber can be mounted on a conveyor belt or a rail or any equivalent suitable device. In another embodiment, automated guided vehicles are provided, to automatically carry the mating chamber from one location to the following.

In one embodiment, the mating chamber is provided with a hook 13 or any suitable means, so that it can be moved automatically in a suspended monorail conveyor. This disposition makes it possible to leave the floor space to be left free for other operations, such as maintenance, surveillance, or even other life stages.

This disposition makes it also possible to control the air flux in the mating cage.

In one embodiment, the air flux is not too high, so as not to inhibit mating by dispersing the pheromones outside the mating cage 1 and complicating the mating dance for insects as clumsy as the Black Soldier Fly.

In one embodiment, step 1 comprises a sub-step of connection with one emergence chamber. By emergence chamber, a skilled man will understand any container suitable for the emergence of the imago, and possibly part or all of the pupal stage. Such an emergence cage comprises a door or a hatch, the closing and opening of which is automated, and that can be connected to the mating cage.

The mating cage also comprises at least one door or hatch 12, for example a sliding door or hatch, the closing and opening of which is automated, and through which the mating cage 1 can be connected to an emergence cage.

The door 12 can be, in an exemplary embodiment, positioned on the underside or on the side faces of the mating cage 1.

In one embodiment, if the mating cage 1 is suspended, filling of the mating cage can be performed through a hatch on the underside of the mating cage 1, with the emerging boxing being moved from one location to another at a lower floor than the mating boxes.

At the beginning of step 1, the door 12 of a mating cage and the one of an emergence cage containing newly emerged imagoes are tightly placed face to face, so that no adult can escape from the assembly formed by the two boxes. For example, the frames of the two doors can be directly clipped to each other. The doors are simultaneously or successively opened so as to allow the newly emerged adults to pass from of the emergence box into the mating cage, for example in response to a stimulus, like a light stimulus.

The size and other characteristics of the pathway between the emergence box and the mating box can be chosen so that only capable adults can reach the mating box.

In one embodiment, only adults able to fly can reach the mating box from the emergence box. Adults that are still incapable to fly will remain in the emergence box and will either be killed when the emergence box is emptied before entering a new emergence cycle, or be allowed to fly into another mating cage to which the emergence box is later connected. This disposition contributes to an even selection of high quality adults (approximately same age or development stage, no disease nor defect, ...). In other words, the adults entering the mating cage are synchronous at the beginning of the mating cycle and ready for mating. This disposition enables a minimum duration of the mating cycle.

The movement of insects can be promoted by different means, for example chemical means or more intense lighting in the mating cage than in the emergence box.

The parameters chosen to promote the movement of insects toward the mating cage also allows for selection of the population according to its phenotype (size, form of the wings,...).

Once the wished population has entered the mating cage and/or after a predefined connection time, the door 12 and the door of the emergence box are automatically closed, either simultaneously or successively.

The count of the population in the mating cage can be automated.

For example, the count of the population is based on the detection that the flies cross a light beam between the emergence box and the mating cage 1.

In case the mating cage is mounted on a suspended rail, the hook 13 can be provided with a weight sensor. The closing of the door 12 can then be controlled by the weight of the cage.

The estimated population in the cage may be estimated from the weight of the mating cage, the known average weight of an insect at emergence stage, and a known ratio of flying time for an insect.

The mating cage 1 can also be weighed in darkness, so that no fly is flying when the measurement is taken.

The mating cage can be designed to promote mating : for a given volume, its height can be up to 2 , 3, 5, 7 or 10 times its length or width so that the height is sufficient for the mating dance.

In a particular embodiment, a given mating cage 1 can be connected successively to several emergence boxes. This disposition makes it possible to mix several larval populations, in order to select or mix particular genotypes or to limit the drifts associated with the rapid life cycle of the insects, such as the selection and reproduction of an unwanted genetic heritage. Consequently, the filling of a mating chamber is relatively reproducible in terms of population characteristics, which allows a better control of the quality of the products associated with the breeding, such as foodstuff, lipids or any commercially valuable byproduct.

The sex ratio can for example be controlled, insofar as the pupal stage of a male is different than that of a female. In the case of *Hermetica Illucens*, the pupal stage of a male is about two days shorter than that of a female. In average, if males emerge in a given emergence box at day d, females will emerge at day d+2. Hence, one given mating cage can be connected with a first emergence box being at the stage corresponding to day d and with a second emergence box being at the stage corresponding to day d+2. More precise distribution may be controlled based on known emergence age curves for the insect.

The duration of the first and the second connection can be set so that at the end of the filling, the female-to-male ratio is around 51% to 65%. This disposition increases the yield of the mating cycle in terms of eggs/cycle.

Other techniques, based on sexual dimorphism, can be considered to control the sex ratio.

Other selection operations may be carried out at the larval stage. For example, the larvae may be sieved in order to select larvae having the same size, and therefore of the same age (or at least the same stage of development).

Other selection operations may be carried out on the basis of the genotype. For example, the eggs of the first generation can be selected on genotypic criteria.

One understands as a consequence that one mating cage can be connected successively to several emergence cages and that reciprocally, an emergence cage can be connected successively to several mating cages.

Typically, the total time (T1) for the filling of the mating cage ranges from ten minutes to an hour.

Once the wished population has filled the mating cage, they enter step 2, at the same or at a different location, where they are subjected to a first lighting cycle to promote the insects mating. If the second step takes place in another location, the cage is automatically moved from the first to the second location.

A fine tuning of the filling of the mating chamber helps having synchronized adults, that is to say adults having a homogeneous sexual maturity. This arrangement allows a fine tuning of the mating chamber environment at each of the following steps, on the contrary to prior art process, in which some adults mate, oviposit and die continuously in the same box.

In the process described here, each step can be specifically designed for a particular step of the adult life : first, it can focus on mating, then on egg maturation and eventually on oviposition.

The first lighting cycle lasts for 24 hours (time period T2) in an exemplary embodiment.

In general, intense lighting during several hours and darkness during the rest of the 24 hours of the day could be suitable. For example, intense lighting during twelve to fourteen hours (or even sixteen hours), followed by twelve to ten hours (or even eight hours) in total or relative darkness will be suitable.

In another embodiment, the first lighting cycle time period T2 is more or less than 24 hours.

The Photosynthetically Active Radiation can for example range up to 500 µmοl per square meter per second. Such an intense light promotes mating from the first hours after the filling of the mating cage.

At any time during this step, one or more ovipositing devices 2 are attached to the mating cage, preferably on its outer side. For example, one removable ovipositing device 2 can be attached to the cage. The attachment of the ovipositing devices 2 to the mating cage 1 can also be carried out before the filling of the mating cage.

One exemplary embodiment of an ovipositing device 2 is represented on figure 2. Females seek for shady and dry places to oviposit. As a consequence, the ovipositing devices 2 must be of a suitable material and include crevices. The ovipositing devices 2 can for example be made of plastic or wood.

For example, an ovipositing device 2 can be in the form of a plastic or wooden parallelepiped much less thick than long and wide, having a width and a length inferior to those of the side of the mating cage to which it will be attached . Grooves or trenches 21, having a depth of the order of a few millimeters, machined on the side facing the mating cage 1 will provide ideal conditions (shadow, dryness,...) for oviposition. Note that for a better understanding of figure 2, the scale is not correct for the dimensions of the trenches.

The trenches can be designed so that eggs can be collected in an automated way.

The face 22 opposite the trenches is turned toward the outside of the mating cage, so that the females in the mating cage can oviposit in the trenches through the cage wall.

Chemical or biological substances (pheromones, egg debris, volatile compounds typical of the food required for eggs ...) can be added to attract gravid females to the ovipositing devices.

These substances can be placed in specific containers.

Theses substances can also directly impregnate the surface of the ovipositing devices 2.

The ovipositing devices 2 can be collected manually or in an automated way.

Therefore, for example, the ovipositing devices 2 can slide between two horizontal rails attached to the outer side of the mating cage 1, or the removable attachment can be a mechanical fixing means, like a bayonet attachment of the ovipositing devices 2 to the mating cage 1.

In the case the ovipositing devices 2 are on the outer face of the mating cage 1, unlike usual practice, no aperture needs to be opened when these devices are collected. As a consequence, no adult can be lost.

However, there is in general no need to collect the ovipositing devices during step 2, since few ovipositing occur at this step. This embodiment will be of greater interest at step 3, 4 or 4'.

For the same reasons, only few ovipositing devices 2 per mating chamber are required at this step.

The mating cage then enters the third step. If this step takes place in another location, the cage is automatically moved from the second to the third location.

The third step comprises a second lighting cycle, in order to give the females a second chance of mating, and that also promotes oocytes maturation.

The ovipositing devices 2 can be collected during or at the end of step 3. When ovipositing devices 2 are collected, new ovipositing devices 2 are provided. Additional ovipositing devices 2 can also be provided during step 3.

In a particular embodiment, the duration T2b of step 3 is the same as T2.

In general, bright lighting during several hours and darkness during the rest of the 24 hours of the day could be suitable. For example, intense lighting during twelve to fourteen hours (or even sixteen hours), followed by twelve to ten hours (or even eight hours) in total or relative darkness will be suitable.

The mating cage then enters the fourth step. If this step takes place in another location, the cage is automatically moved from the third to the fourth location. During the fourth step, the mating cage undergoes a third lighting cycle (time period T6) that promotes oocytes maturation in the gravid females and oviposition.

Preferably, the light intensity is reduced compared to step 2 and/or step 3, since females require (and search for) shadow places for ovipositing. This disposition is also associated with energy saving.

In an exemplary embodiment, the time period T6 is a 24-hours day

In general, intense lighting during several hours and darkness during the rest of the 24 hours of the day could be suitable. For example, intense lighting during twelve to fourteen hours (or even sixteen hours), followed by twelve to ten hours (or even eight hours) in total or relative darkness will be suitable.

New ovipositing devices are attached to the mating cage at the beginning of step 4. More ovipositing devices than in step 2 and/or step 3 can be provided, in so far as step 4 corresponds to the day of peak egg production.

At least some of the ovipositing devices 2 are manually or automatically collected at least at the end of step 4. They can be collected at shorter time intervals, for example if they are satisfactorily filled. For example, the intensity of a light beam having the same direction as one or more trenches before entering and after crossing the one or more trenches can be compared in order to assess the filling of the trenches.

Once the eggs are collected, they can immediately or later enter an egg maturation cycle that will not be described here.

The mating cage then optionally enters step 4', which can be similar to step 4. If this step takes place in another location, the cage is automatically moved from the fourth to the next location. During step 4', the mating cage undergoes a fourth lighting cycle that promotes oviposition.

Preferably, the light is less intense than in steps 2 and 3.

In an exemplary embodiment, the duration (or time period) T6b of step 4' is 24 hours.

In general, moderate lighting during several hours and darkness during the rest of the 24 hours of the day could be suitable. For example, intense lighting during twelve to fourteen hours (or even sixteen hours), followed by twelve to ten hours (or even eight hours) in total or relative darkness will be suitable.

At this stage, ovipositing devices 2 are attached to the mating cage, on its outer side. For example two or three removable ovipositing devices can be attached to the cage.

The ovipositing devices are manually or automatically collected at least at the end of step 4'. They can be collected at shorter time intervals, for example if they are satisfactorily filled.

Once the eggs are collected, they can enter an egg maturation cycle that will not be described here.

The mating cage then enters the fifth step. If this step takes place in another location, the cage is automatically moved from the current to the next location. During the fifth step, ovipositing is stopped. This means that the adults no longer have the opportunity to oviposit and no ovipositing device is collected after the beginning of step 5.

In one embodiment, the adults still alive at the beginning of step 5 are killed before their natural death.

For example, step 5 takes place at the end of day 4 after the beginning of step 1. This implies, in the case of Black Soldier Flies, that most of the adults are killed before their natural death (which occurs in general seven to ten days after emergence). In other words, step 5 takes place after a duration less than the adult life expectancy after the beginning of step 1.

The adult life expectancy must be understood as the average duration between emergence and natural death of an insect of a given species.

At the beginning of step 5, some of the adults are already dead, in particular some of the females who have already oviposited, but some of them are still alive.

One aspect is to consider that to optimize the egg yield, one has to shorten the time spent by the adults in the mating cage 1, on the contrary to usual practice, which consists in waiting for the death of all the adults in a cage.

One will also remember that a female mates at most once. All the eggs that are collected correspond to a mating during days 1, 2 or 3 after the beginning of step 1 if step 5 takes place at the end of day 4.

Liquid or gaseous fluid, for example water, can be projected in the mating cage by means of nozzles, so as to drown the insects.

Water vapor is preferred since it makes this step faster and allows simultaneous disinfection of the cage. In this case, the mating cage can be made of a heat resistant mesh such as stainless steel mesh.

In another embodiment, at step 5, the mating cage can be connected to another cage and the adults directed to this cage by means of intense lighting in this other cage, while the mating cage is kept in the dark. Once the mating cage 1 is empty, that is to say contains no living adult, it is disconnected from the other cage.

In general, any means which makes it possible to significantly reduce the mating rate and the oviposition rate compared to the rates observed on average under optimal conditions on the date considered after emergence is suitable for the achievement of step 5.

The decision to start step 5 can be made on several basis :
- Either counting the dead adults. When the percentage of dead adults meets a first threshold, step 5 begins.
- Or counting the females having oviposited. When the percentage of females having oviposited meets a second threshold, step 5 begins.
- Or counting the number of eggs collected, or weighing the eggs collected.When the number or the weigh of eggs collected meets a third threshold, step 5 begins.

Step 5 doesn't necessarily start as soon as one of the criteria is met. It can start as soon as possible, depending for example on the position of other mating cages 1 in the system.

Maintaining darkness in the mating cage, decreasing the temperature, the lack of oviposition devices, ..., can thus be implemented in particular embodiments.

The duration (T3) of step 5 can be less than the durations of any of step 2, 3 4 or 4'. For example, T3 is less than one hour.

The fifth step guarantees that the collected eggs have been oviposited after a mating occurring during the first two days of the female lives. This step provides as a consequence an enhanced control of the quality and characteristics of the eggs collected, and thus a better synchronization of the insects entering the larval stage and, later, a new mating cycle.

In the hypothesis where the mating cycle is coupled with an egg maturation cycle and a larval cycle, after a few cycles corresponding to a transient state, one may consider that synchronization is optimal and the three cycles combined have steady-state production.

As a result, organoleptic qualities or other characteristics of the products of the insects rearing can be relatively stable from batch to batch.

Since not all the adults die before day 5, the precocious termination of the adult phase (that is to say the phase during which the eggs resulting from mating and ovipositing are collected) also limits ovipositing on dead adults, which would result in a lower global egg yield.

In this document, the egg yield is the mass of eggs collected par cubic meter of mating chamber per time unit.

The mating cage then enters the sixth step. If this step takes place in another location, the cage is automatically moved from the current to the next location.

At this step, the mating cage is cleaned, optionally sterilized, and dried, so as to be reusable for a new step 1.

In the case water vapor is projected at step 5, this enables step 6 to take place at the same location and at the same time as step 5. In this case, cleaning the mating cage 1 means also sterilizing this mating cage.

In case the sliding door 12 is on the underside of the mating cage, it can be reopened at this step to remove debris.

One or more additional steps can be included in the mating cycle.

In another example, step 4 may be repeated one or several times, as a function of the adult life time of the insect bred and the duration of the optimal period for mating at the beginning of the adult stage.

The main limitation is that the adults must be killed before their natural deaths to prevent late mating, late ovipositing, corresponding to poorer egg quality.

In another embodiment, one or more storage steps can be included in the mating cycle.

After the last step, the mating cage starts a new cycle, in step 1. If the mating cage was displaced from step to step, there is no need to remove the cage from the conveyor belt (or rail,...) between two successive cycles.

For Black Soldier Flies, it has been observed that performing step 5 four days after filling of the mating cage results in 100% fertilized eggs entering the larval stage.

With a cycle as described on Fig. 3, yields of 10 g eggs per cubic meter of mating cage per day or more have been observed.

The temperature and/or relative humidity can be adjusted at each step. In one embodiment, in the case of Black Soldier Flies, the temperature for all the steps between step 1 and step 5 is around 30°C, preferably in the range [25°C, 35°C], and the relative humidity is around 80% for all these steps.

The lighting can be adapted for each step. For example, the spectrum of the light for any of the steps 2 to 4' may include one or more monochromatic radiations selected from the UV spectrum and/or the visible spectrum. The Photosynthetically Active Radiation can be chosen in the range 80 µmol/m²/s to 500 µmol/m²/s.

Feeding or watering can be provided at one or more steps.

The process which has just been described may be performed in an automated installation.

The automated installation may comprise a plurality of stations each adapted to perform a specific step. According to one example, the stations are provided along a predefined path. The path is cyclic. The automated installation comprises one or more mating cages 1, which undergo the above process cyclically.

A controller system is provided to control the operation of the automated installation. The controller system is adapted to control the cyclic displacement of the mating cages along the plurality of stations. For example, the automated installation comprises a conveyor system to guide the movement of the mating cages along the path. In case of a plurality of mating cages, these may be handled synchronously, in a time-shifted manner, so that, at a given time, mating cages are located in various stations, and undergo different steps of the above process.

Stations may be closed, or partially closed, in order to control the ambient conditions (in particular, temperature, humidity, lighting) in the station when necessary. "Closed" here should be understood that the closure enables the condition to be maintained in the station, while being different outside the station, while still not preventing the mating cage from entering or exiting the station.

At each station, a mating cage 1 may be still. Then, the controller system controls the shifting of the mating cage to a next station based on a predefined criterion, such as a predefined schedule and/or a sensed characteristic meeting a predefined condition. In addition, or alternatively, the controller system may control a continuous movement of the mating cage along the path in one or more of the stations.

To allow the controller system to track the mating cages 1, in a particular embodiment, a tracking device can be provided for each mating cage 1. For example, the tracking device is a RFID chip or a barcode.

This tracking device is of particular interest for the filling of a mating cage 1, especially when it is filled with adults from different emergence chambers. For example, it helps monitor the successive connections between one or more mating cage 1 and one or more emergence chambers. As a consequence, it enables an automated population selection for any mating cage.

At each station, the controller system may control the operation of the station. This may involve triggering any action based on a predefined criterion, such as a predefined schedule and/or a sensed characteristic meeting a predefined condition. The controller system may therefore be programmed to cause a movement (opening or closing a door, attaching or removing a part) and/or modifying an operational condition (switching lights on or off, modifying ambient temperature, ...).

A given mating cage 1 being ready for entering a new cycle after step 6 (or optionally step 6b), an automated installation for the imaginal stage cycle can comprise several mating cages 1, one or more mating cages 1 being at one or more station at the same time. A given mating cage 1 periodically runs the imaginal stage cycle over a period called Tcycle.

The controller system may further control other cycles of the installation than the imaginal stage cycle of the mating cages 1, in synchronism with the imaginal stage cycle of mating cages. One particular example is a cycle of emergence cages, which can be controlled by the controller system and synchronized with the cycle of the mating cage.

Another example is a cycle of ovipositing devices, which can be handled in synchronicity with the mating cages to be assembled to and disassembled from the mating cages, and automatically treated for harvesting laid eggs.

In one embodiment, a tracking device can be provided for each oviposited device 2. For example, the tracking device is an RFID chip or a barcode.

Although particular embodiments of the invention have been disclosed herein in detail, this has been done by way of example and for the purposes of illustration only. The aforementioned embodiments are not intended to be limiting with respect to the scope of the invention. It is contemplated by the inventors that various substitutions, alterations, and modifications may be made to the invention without departing from the scope of the investion as defined by the appended claims.

### LIST OF THE REFERENCE SIGNS

1 : mating cage
11 : mesh
12 door
13 hook
2 : ovipositing device
21 : trench for ovipositing
22 : face of the ovipositing device opposite the trenches
3: exemplary mating cycle

## Claims

1. Automated system for breeding insects at their imaginal stage, comprising :
- at least one mating cage (1) having at least one door or hatch (12) the closing and opening of which is automated and through which said mating cage (1) can be connected to at least one emergence chamber having at least one door or hatch the closing and opening of which is automated;
- a filling system adapted to fill the at least one mating cage (1) with a population of newly emerged insects during a first time interval T1 and to automatically close successively or simultaneously the at least one door or hatch of the mating cage and the at least one door or hatch of the at least one emergence chamber when connected to the at least one mating cage (1) once a wished population has entered the mating cage and or/after a predefined connection time;
- a provision system adapted to provide conditions within the at least one mating cage (1) suitable for promoting mating and/or oocytes maturation and/or ovipositing during a second time interval T2;
- a terminating system adapted to stop ovipositing during a third time interval T3, the time between the beginning of a filling of the at least one mating cage (1) and the beginning of said stopping of the oviposition in said at least one mating cage (1) being shorter than the life expectancy of the adult insects after emergence.

2. An automated system according to claim 1, wherein
a. said at least one mating cage (1)
- comprises an automated device for closing and opening said at least one door hatch (12),
- is provided with at least one removable ovipositing device (2), and
- has at least two discrete positions, and,
b. said automated system further comprises a device for moving said at least one mating cage (1) from at least one of the said at least two discrete positions to at least another of the said at least two discrete positions, each discrete position being equipped to automatically perform at least one step chosen among :
- connecting said at least one mating cage (1) through its at least one door or hatch (12) to at least one emergence chamber comprising newly emerged insects and having a door or a hatch the closing and opening of which is automated and filling said at least one mating cage (1) with said filling system;
- filling said at least one mating cage (1) with said population of newly emerged insects with said filling system;
- providing said conditions suitable for promoting mating and/or oocytes maturation and/or ovipositing within said at least one mating cage (1) with said provision system
- in a mating cage (1) that has been filled with a population of newly emerged insects, stopping ovipositing with said terminating system.

3. Automated system according to claim 2 **characterized in that** each of said at least one mating cage (1) is provided with an automated device for counting the adult population and **in that** said automated device for closing and opening said door or hatch (12) of the at least one mating cage (1) receives a signal from said automated device for counting the adult population that triggers the closing and/or opening of said door or hatch (12).

4. Automated system according to claim 2 or claim 3 further comprising a controller and wherein a tracking device is provided for each of said at least one mating cage (1), the controller receiving tracking data from said at least one mating cage (1) and controlling the moving of the at least one mating cage (1) based on the tracking data.

5. Automated system according to any of claims 2 to 4 **characterized in that** at least one removable ovipositing device (2) is provided for each mating cage (1) and that said removable ovipositing device (2) is fixed on the outer side of said mating cage (1) and is provided with a tracking device, the system further comprising a controller receiving data from at least one tracking device of an ovipositing device (2) and controlling the removal and the fixing of the ovipositing device (2) based on the tracking data received from the ovipositing device (2).

6. A system comprising a system according to any of claims 1 to 5 coupled with a system for breeding insects at the egg stage and/or at the pupation stage.

## Patentansprüche

1. Automatisiertes System zur Züchtung von Insekten in ihrem Imaginalstadium, das Folgendes umfasst:
- wenigstens einen Paarungskäfig (1) mit wenigstens einer Tür oder Luke (12), deren Schließen und Öffnen automatisiert ist und durch die der genannte Paarungskäfig (1) mit wenigstens einer Schlüpfkammer mit wenigstens einer Tür oder Luke, deren Schließen und Öffnen automatisiert ist, verbunden werden kann;
- ein Füllsystem, das so gestaltet ist, dass der wenigstens eine Paarungskäfig (1) mit einer Population frisch geschlüpfter Insekten während eines ersten Zeitintervalls T1 befüllt und die wenigstens eine Tür oder Luke des Paarungskäfigs und die wenigstens eine Tür oder Luke der wenigstens einen Schlüpfkammer, wenn sie mit dem wenigstens einen Paarungskäfig (1) verbunden ist, automatisch nacheinander oder gleichzeitig geschlossen werden, nachdem eine gewünschte Population in den Paarungskäfig eingetreten ist und/oder nach einer vordefinierten Verbindungszeit;
- ein Bereitstellungssystem zum Bereitstellen von Bedingungen innerhalb des wenigstens einen Paarungskäfigs (1), die zum Fördern der Paarung und/oder der Eizellreifung und/oder der Eiablage während eines zweiten Zeitintervalls T2 geeignet sind;
- ein Beendigungssystem zum Stoppen der Eiablage während eines dritten Zeitintervalls T3, wobei die Zeit zwischen dem Beginn des Befüllens des wenigstens einen Paarungskäfigs (1) und dem Beginn des genannten Stoppens der Eiablage in dem genannten wenigstens einen Paarungskäfig (1) kürzer als die Lebenserwartung der adulten Insekten nach dem Schlüpfen ist.

2. Automatisiertes System nach Anspruch 1, wobei
a. der genannte wenigstens eine Paarungskäfig (1)
- eine automatisierte Vorrichtung zum Schließen und Öffnen der genannten wenigstens einen Tür oder Luke (12) umfasst,
- mit wenigstens einer entfernbaren Eiablagevorrichtung (2) versehen ist, und
- wenigstens zwei diskrete Positionen hat, und
b. das genannte automatisierte System ferner eine Vorrichtung zum Bewegen des genannten wenigstens einen Paarungskäfigs (1) von wenigstens einer der genannten wenigstens zwei diskreten Positionen zu wenigstens einer anderen der genannten wenigstens zwei diskreten Positionen umfasst, wobei jede diskrete Position so ausgelegt ist, dass wenigstens ein Schritt automatisch ausgeführt wird, ausgewählt aus:
- Verbinden des mindestens einen Paarungskäfigs (1) durch seine mindestens eine Tür oder Luke (12) mit mindestens einer Emergenzkammer, die frisch geschlüpfte Insekten enthält und eine Tür oder eine Luke aufweist, deren Schließen und Öffnen automatisiert ist, und Füllen des mindestens einen Begattungskäfigs (1) mit dem Füllsystem;
- Befüllen des genannten wenigstens einen Paarungskäfigs (1) mit der genannten Population frisch geschlüpfter Insekten mit dem genannten Füllsystem;
- Bereitstellen der genannten Bedingungen, die zum Fördern der Paarung und/oder der Eizellreifung und/oder der Eiablage innerhalb des genannten wenigstens einen Paarungskäfigs (1) geeignet sind, mit dem genannten Bereitstellungssystem;
- in einem Paarungskäfig (1), der mit einer Population frisch geschlüpfter Insekten gefüllt ist, die Eiablage mit dem genannten Abschlusssystem zu stoppen.

3. Automatisiertes System nach Anspruch 2, **dadurch gekennzeichnet, dass** jeder der genannten wenigstens einen Paarungskäfige (1) mit einer automatisierten Vorrichtung zum Zählen der adulten Population versehen ist und dass die genannte automatisierte Vorrichtung zum Schließen und Öffnen der genannten Tür oder Luke (12) des wenigstens einen Paarungskäfigs (1) ein Signal von der genannten automatisierten Vorrichtung zum Zählen der adulten Population empfängt, das das Schließen und/oder Öffnen der genannten Tür oder Luke (12) auslöst.

4. Automatisiertes System nach Anspruch 2 oder Anspruch 3, das ferner eine Steuerung umfasst und wobei eine Trackingvorrichtung für jeden der genannten wenigstens einen Paarungskäfige (1) vorgesehen ist, wobei die Steuerung Trackingdaten von dem genannten wenigstens einen Paarungskäfig (1) empfängt und die Bewegung des wenigstens einen Paarungskäfigs (1) anhand der Trackingdaten steuert.

5. Automatisiertes System nach einem der Ansprüche 2 bis 4, **dadurch gekennzeichnet, dass** für jeden Paarungskäfig (1) wenigstens eine entfernbare Eiablagevorrichtung (2) vorgesehen ist und dass die genannte entfernbare Eiablagevorrichtung (2) an der Außenseite des genannten Paarungskäfigs (1) befestigt und mit einer Trackingvorrichtung versehen ist, wobei das System ferner eine Steuerung umfasst, die Daten von wenigstens einer Trackingvorrichtung einer Eiablagevorrichtung (2) empfängt und das Entfernen und Befestigen der Eiablagevorrichtung (2) anhand der von der Eiablagevorrichtung (2) empfangenen Trackingdaten steuert.

6. System, das ein System nach einem der Ansprüche 1 bis 5 umfasst, das mit einem System zur Züchtung von Insekten im Eistadium und/oder im Verpuppungsstadium gekoppelt ist.

## Revendications

1. Système automatisé d'élevage d'insectes à leur stade imaginal, comprenant :
- au moins une cage d'accouplement (1) comportant au moins une porte ou trappe (12) dont la fermeture et l'ouverture sont automatisées et par laquelle ladite cage d'accouplement (1) peut être reliée à au moins une chambre d'émergence comportant au moins une porte ou trappe dont la fermeture et l'ouverture sont automatisées ;
- un système de remplissage adapté pour remplir la au moins une cage d'accouplement (1) avec une population d'insectes nouvellement émergés pendant un premier intervalle de temps T1 et pour fermer automatiquement successivement ou simultanément la au moins une porte ou trappe de la cage d'accouplement et la au moins une porte ou trappe de la au moins une chambre d'émergence lorsqu'elle est connectée à la au moins une cage d'accouplement une fois qu'une population souhaitée est entrée dans la cage d'accouplement et/ou après un temps de connexion prédéfini ;
- un système de mise à disposition adapté pour fournir des conditions à l'intérieur de la au moins une cage d'accouplement (1) adaptées pour favoriser l'accouplement et/ou la maturation et/ou la ponte pendant un deuxième intervalle de temps T2 ;
- un système d'arrêt adapté pour arrêter la ponte pendant un troisième intervalle de temps T3, le temps entre le début d'un remplissage de la au moins une cage de reproduction (1) et le début dudit arrêt de la ponte dans ladite au moins une cage de reproduction (1) étant plus court que l'espérance de vie des insectes adultes après l'émergence.

2. Système automatisé selon la revendication 1, dans lequel
a. ladite au moins une cage d'accouplement (1)
- comprend un dispositif automatisé pour la fermeture et l'ouverture de ladite au moins une porte ou trappe (12),
- est pourvu d'au moins un dispositif de ponte amovible (2), et
- a au moins deux positions discrètes, et,
b. ledit système automatisé comprend en outre un dispositif pour déplacer ladite au moins une cage d'accouplement (1) d'au moins une desdites au moins deux positions discrètes à au moins une autre desdites au moins deux positions discrètes, chaque position discrète étant équipée pour automatiquement effectuer au moins une étape choisie parmi :
- connecter ladite au moins une cage d'accouplement (1), par l'intermédiaire de sa au moins une porte ou une trappe (12), à au moins une chambre d'émergence comprenant des insectes nouvellement émergés et dotée d'une porte ou d'une trappe dont la fermeture et l'ouverture sont automatisées, et remplir ladite au moins une cage d'accouplement (1) à l'aide dudit système de remplissage ;
- remplir ladite au moins une cage d'accouplement (1) avec ladite population d'insectes nouvellement émergés avec ledit système de remplissage ;
- fournir lesdites conditions appropriées pour favoriser l'accouplement et/ou la maturation et/ou la ponte à l'intérieur de ladite au moins une cage d'accouplement (1) avec ledit système de fourniture ;
- dans une cage d'accouplement (1) qui a été remplie d'une population d'insectes nouvellement émergés, arrêter la ponte à l'aide dudit système d'arrêt.

3. Système automatisé selon la revendication 2 **caractérisé en ce que** chacune de ladite au moins une cage d'accouplement (1) est munie d'un dispositif automatisé de comptage de la population adulte et **en ce que** ledit dispositif automatisé de fermeture et d'ouverture de ladite porte ou trappe (12) de la au moins une cage d'accouplement (1) reçoit un signal dudit dispositif automatisé de comptage de la population adulte qui déclenche la fermeture et/ou l'ouverture de ladite porte ou trappe (12).

4. Système automatisé selon la revendication 2 ou la revendication 3 comprenant en outre un contrôleur et dans lequel un dispositif de suivi est prévu pour chacune de ladite au moins une cage d'accouplement (1), le contrôleur recevant des données de suivi de ladite au moins une cage d'accouplement ( 1) et commandant le déplacement de la au moins une cage d'accouplement (1) sur la base des données de suivi.

5. Système automatisé selon l'une quelconque des revendications 2 à 4 **caractérisé en ce qu'**au moins un dispositif de ponte amovible (2) est prévu pour chaque cage d'accouplement (1) et que ledit dispositif de ponte amovible (2) est fixé sur le côté extérieur de ladite cage d'accouplement (1) et est pourvu d'un dispositif de suivi, le système comprenant en outre un contrôleur recevant des données d'au moins un dispositif de suivi d'un dispositif de ponte (2) et commandant le retrait et la fixation du dispositif de ponte (2) sur la base de données de suivi reçues du dispositif de ponte (2).

6. Système comprenant un système selon l'une quelconque des revendications 1 à 5 couplé à un système d'élevage d'insectes au stade oeuf et/ou au stade nymphose.
